# EUROPEAN PATENT APPLICATION

(11) **EP 4 706 752 A1**
(43) Date of publication of application: **11.03.2026**
(21) Application number: 24209616.2
(22) Date of filing: 29.10.2024
(51) Int. Cl.: A61N 5/06, H10K 50/87, A61N 5/00, H10K 102/00

(54) **OLED DEVICES HAVING COOLING SYSTEM AND METHOD FOR DRIVING THEREOF**

(30) Priority: 06.09.2024 KR 20240121456; 15.10.2024 KR 20240140095
(71) Applicant: DSLAB, Inc., Suwon-si, Gyeonggi-do 16648 (KR)
(72) Inventor: JO, Deok Su, 16551 Gyeonggi-do (KR); LEE, Weonseok, 16358 Gyeonggi-do (KR); KIM, Ji Eun, 16548 Gyeonggi-do (KR); TRAN, Thien Tri, 16417 Gyeonggi-do (KR)
(74) Representative: Lippert Stachow Patentanwälte Rechtsanwälte Partnerschaft mbB

(57) **Abstract**

The present disclosure relates to an OLED device (10) with cooling system (20) and driving method thereof, and the OLED device according to one embodiment of the present disclosure includes an OLED, a cooling system including a cooling element for cooling the OLED, a temperature sensor (30) for measuring a temperature of the OLED or of an area adjacent to the OLED, and a control unit (40) for controlling driving of the OLED and of the cooling system, wherein the control unit drives the cooling system prior to driving the OLED, and controls such that the OLED starts to be driven when the temperature measured by the temperature sensor is at or below a preset temperature.

## Description

### Technical Field

The present disclosure relates to an OLED device and driving method thereof. More specifically, the present disclosure relates to an OLED device with improved electrical and optical characteristics by utilizing a cooling system, and to a method for driving the same.

### Background Art

Phototherapy is a treatment method that uses light of specific wavelengths to treat various medical conditions or alleviate symptoms.

Depending on the therapeutic purpose and the targeted disease, ultraviolet (UV) light sources, visible light sources, infrared (IR) light sources, laser light sources, fluorescent lamps, LED light sources, and OLED light sources are being used in phototherapy.

Among these, fluorescent lamps, LED light sources, and OLED light sources are relatively inexpensive and safe to be used by the general public, allowing them to be used effectively in phototherapy devices used in households.

However, increasing the applied power to achieve sufficient light output for phototherapy may cause side effects, such as burns, tissue deformation and the like due to the high heat generated from the light source.

Additionally, when using a light conversion film (for example, converting the red light of a Red OLED to near-infrared (NIR)), the light output is reduced by one-third to one-half, and thus it is necessary to increase the applied power to achieve sufficient light output. However, if the power applied to the light source is too high, the light source may become damaged, leading to a problem known as light quenching phenomenon, where light output is reduced.

[Prior Art Document] Korean Patent Registration No. 10-2382643

### Disclosure

### Technical Problem

A purpose of the present disclosure is to provide an OLED device with cooling system and driving method thereof.

Another purpose of the present disclosure is to provide an OLED device suitable for phototherapy and driving method thereof.

Another purpose of the present disclosure is to provide an OLED device that can provide high light output even at temperatures lower than body temperature and driving method thereof.

Another purpose of the present disclosure is to provide an OLED device where temperature and light output increase consistently according to input power and driving method thereof.

Another purpose of the present disclosure is to provide an OLED device with flexibility that is suitable for use on curved human body parts and driving method thereof.

### Technical Solution

The above and other purposes of the present disclosure can all be achieved by the OLED device with cooling system and driving method thereof according to the present disclosure.

An OLED device according to one embodiment of the present disclosure includes an OLED, a cooling system including a cooling element for cooling the OLED, a temperature sensor for measuring a temperature of the OLED or of an area adjacent to the OLED, and a control unit for controlling driving of the OLED and of the cooling system.

The control unit drives the cooling system prior to driving the OLED, and controls such that the OLED starts to be driven when the temperature measured by the temperature sensor is at or below a preset cooling temperature.

Furthermore, the OLED is a flexible OLED, and the cooling system may be configured to include a plurality of cooling elements arranged to be spaced apart from each other, so that the OLED device according to one embodiment of the present disclosure has flexibility.

The cooling element is a Peltier element, and the cooling system further includes a heat sink and a fan for dissipating heat generated in the cooling element.

Furthermore, the OLED includes one OLED unit element or a stack of a plurality of OLED unit elements between a substrate located at the cooling element's side and a substrate located at a light-emitting surface's side, and the upper substrate may be a metal substrate or a substrate having a metal film layer.

In addition, the cooling temperature may be a temperature at which light with a saturated power density can be output without malfunction when the OLED is driven after being cooled to the cooling temperature.

In addition, the cooling temperature may be a temperature that ensures the temperature measured by the temperature sensor when the OLED outputs the light with the saturated power density is not heated to a temperature above body temperature or protein denaturation temperature.

The cooling temperature may be 5°C or below, or 0°C or below.

The control unit applies power to the OLED within a preset maximum power range, and the maximum power may be a power at which the OLED outputs the light with the saturated power density when applied to the OLED.

The control unit may control to lower the power applied to the OLED or stop the driving of the OLED if the temperature measured by the temperature sensor is a higher temperature than body temperature or protein denaturation temperature.

The cooling temperature is -15°C to 0°C, and the control unit may control such that the temperature measured by the temperature sensor after the OLED is driven maintains a range of 0°C to 5°C.

A driving method of an OLED device according to one embodiment of the present disclosure includes driving a cooling system to cool an OLED; measuring a temperature of the OLED or of an area adjacent to the OLED; and starting to drive the OLED if the measured temperature is at or below a preset cooling temperature.

The cooling temperature may be a temperature at which light with a saturated power density can be output without malfunction when the OLED is driven after being cooled to the cooling temperature.

Furthermore, the cooling temperature may be a temperature that ensures the temperature measured by the temperature sensor when the OLED outputs the light with the saturated power density is not heated to a temperature above body temperature or protein denaturation temperature.

The driving method of the OLED device according to one embodiment of the present disclosure may further include lowering the power applied to the OLED or stopping the driving of the OLED if the temperature measured by the temperature sensor after the OLED is driven is higher than body temperature or protein denaturation temperature.

The cooling temperature may be 5°C or below, or 0°C or below.

Furthermore, the driving method of the OLED device according to one embodiment of the present disclosure may be characterized in that the cooling temperature is -15°C to 0°C, and may further include controlling such that the temperature measured by the temperature sensor after the OLED is driven maintains a range of 0°C to 5°C.

### Advantageous Effects

The OLED device and driving method thereof according to the present disclosure can provide high light output even at low temperatures, thereby offering a high phototherapy effect without the risk of skin damage and also providing a beneficial effect for pruritus.

Additionally, the OLED device and driving method thereof according to the present disclosure can provide the effect of allowing users to easily adjust the desired temperature and light output, as the temperature and light output increase consistently with an increase in input power.

Furthermore, the OLED device and driving method thereof according to the present disclosure can provide the effect of enabling close contact with curved human body parts due to is flexibility.

Moreover, the OLED device and driving method thereof according to the present disclosure can provide the effect of improving the light output efficiency of fluorescent OLEDs.

### BRIEF DESCRIPTION OF THE DRAWINGS

FIG. 1 is a schematic diagram of an OLED device according to one embodiment of the present disclosure.
FIG. 2 is a schematic diagram of an OLED device according to another embodiment of the present disclosure.
FIG. 3 is a flowchart of a driving method of an OLED device according to the present disclosure.
FIG. 4 is a schematic diagram of an OLED device according to another embodiment of the present disclosure.
FIG. 5 is a graph comparing the temperature of an OLED and light power density according to input power and power consumption when the OLED in Example 1 is driven without a cooling system and when it is driven after being cooled to 0°C using a cooling system.
FIG. 6 is a graph showing the irradiance according to input power and the irradiance according to power consumption when the OLED in Example 1 was driven simultaneously with a cooling system.
FIG. 7 is a graph showing the light power density and temperature of the OLED according to applied power, and the light power density and temperature of the OLED according to power consumption, when the OLED in Example 1 is driven without cooling and after being cooled to 0°C, 5°C, 10°C, 15°C, and 20°C using a cooling system.
FIG. 8 is a graph showing the light power density and temperature of the OLED according to applied power, and the light power density and temperature of the OLED according to power consumption, when the OLED in Example 2 is driven without cooling and after being cooled to 0°C, 5°C, 10°C, 15°C, and 20°C using a cooling system.
FIG. 9 is a graph showing the light power density and temperature of the OLED according to applied power, and the light power density and temperature of the OLED according to power consumption, when the OLED in Example 3 is driven without cooling and after being cooled to 0°C, 5°C, 10°C, 15°C, and 20°C using a cooling system.
FIG. 10 is a graph showing the rate of change in irradiance and current when driving the OLED device according to one embodiment of the present disclosure for 70 minutes continuously and for 12 hours continuously, with the applied power maintained so that a saturated power density is output.

### DETAILED DESCRIPTION

Hereinbelow, an OLED device with cooling system and driving method thereof according to the present disclosure will be described in detail with reference to the attached drawings.

In the following description, only the parts necessary to understand the OLED device with cooling system and driving method thereof according to an embodiment of the present disclosure will be explained, and explanations of other parts may be omitted so as not to deviate from the essence of the present disclosure.

Furthermore, the terms or words used in this specification and the scope of claims described hereinbelow should not be interpreted as being limited to their conventional or dictionary meanings, but rather should be interpreted based on the meanings and concepts consistent with the technical spirit of the present disclosure, so as to most appropriately describe the present disclosure.

Throughout the specification, when a part is described as "including" a specific component, this means that other components may also be included unless explicitly stated otherwise, rather than excluding other components. Also, terms such as "unit," "-er," or "module" as used in the specification refer to a unit that processes at least one function or operation, and this may be implemented as hardware, software, or a combination of hardware and software.

In various embodiments, components with the same configuration will be represented by the same reference numerals and will be explained in detail in a representative embodiment. For other embodiments, only configurations different from those of the representative embodiment will be described.

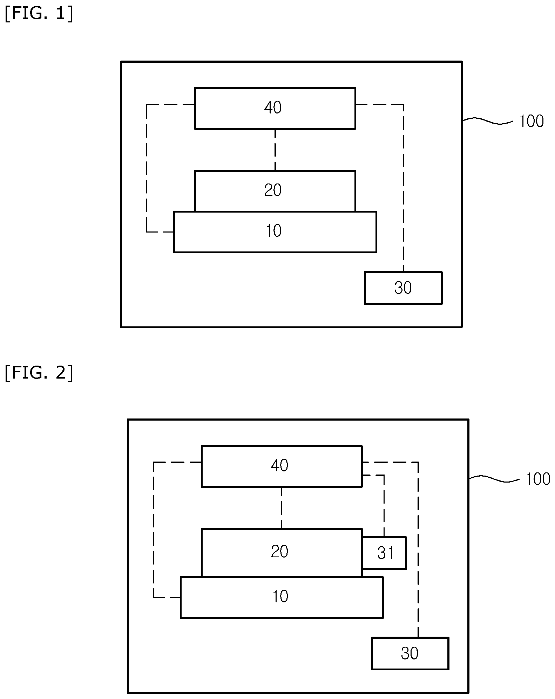

FIG. 1 is a schematic diagram of an OLED device according to one embodiment of the present disclosure.

As illustrated in FIG. 1, an OLED device 100 according to one embodiment of the present disclosure includes an OLED 10, a cooling system 20, a temperature sensor 30, and a control unit 40.

The OLED 10 serves as a light source for the OLED device 100 according to one embodiment of the present disclosure, and may be any type of OLED that provides light, such as a red OLED emitting RED light, a yellow OLED emitting YELLOW light, a green OLED emitting GREEN light, a blue OLED emitting BLUE light, a phosphorescent OLED, and a fluorescent OLED.

In addition, the OLED device 100 according to one embodiment of the present disclosure may be, for example, a phototherapy device, in which case the OLED 10 may be an OLED that provides light with wavelengths required for phototherapy.

The OLED 10 may be configured to include one or more OLED unit elements between an upper substrate and a lower substrate. In this case, a plurality of OLED unit elements may be arranged in a planar configuration, and for higher light output, the plurality of OLED unit elements may be stacked in a tandem structure between the upper substrate and the lower substrate.

Furthermore, in order for the OLED 10 to be efficiently cooled by the cooling system to be described later, it is preferable for the upper substrate, which is located near a cooling element, to be a metal substrate or a substrate with a metal film layer on a plastic substrate.

Additionally, the OLED 10 may include a light conversion film (such as a film that converts emitted light into near-infrared light, etc.).

The cooling system 20 is a system for cooling the OLED 10.

The cooling system is a system that not only dissipates the heat generated as the OLED operates, but it is also capable of cooling the OLED even when it is not generating heat.

The cooling system is a system that cools the OLED, which is at room temperature before operation, to a temperature lower than room temperature, specifically cooling it to 5°C or more below room temperature, preferably to 5°C or below (0°C to 5°C, -15°C to 5°C), and more preferably, to 0°C or below (around 0°C, -15°C to 0°C). Therefore, the cooling system includes a cooling element such as a Peltier element.

At this time, the cooling performance may vary depending on the packaging material of the Peltier element, such as ceramic, metal, or plastic. Thus, the packaging material of the Peltier element can be selected according to the desired cooling performance.

Additionally, the cooling system may further include a heat sink and a fan to dissipate the heat generated by the cooling element.

The temperature sensor 30 is a sensor for measuring the temperature of the OLED or the temperature of an area adjacent to the OLED.

The temperature sensor provides a measured temperature value to the control unit, which will be described later, so that the control unit can control the driving of the OLED 10 based on the measured temperature value.

The temperature measured by the temperature sensor may be the temperature of the OLED surface. Alternatively, the temperature of the area adjacent to the OLED, being close to the OLED, may be considered as the temperature of the OLED, or it may be used to estimate the temperature of the OLED based on the temperature of that area. The OLED device according to one embodiment of the present disclosure is a phototherapy device, and the area adjacent to the OLED may be an area between the OLED and the skin where the OLED's light is irradiated.

Meanwhile, the temperature measured at the surface of the OLED, which is exposed directly to the outside, and the area between the OLED and the skin may be higher than the actual temperature of the OLED due to external influences. Therefore, as shown in FIG. 2, a temperature sensor 31 that can measure the temperature of the OLED surface or the cooling element, which is not directly exposed to the outside, may be added, and the temperature measured by the temperature sensor 31 may be additionally considered to adjust the temperature of the OLED to a desired temperature.

The control unit 40 controls driving of the OLED 10 and the cooling system 20.

Specifically, the control unit drives the OLED and the cooling system such that the cooling system 20 operates together when the OLED 10 operates.

More preferably, the control unit 40 drives the cooling system 20 before driving the OLED 10. Then, when the temperature of the OLED or an area adjacent to the OLED, measured by the temperature sensor 30 reaches a preset cooling temperature or below, the OLED is driven.

In general, if the power applied to the OLED is increased, the OLED is damaged before outputting light with a saturated power density. In contrast, by cooling the OLED to an appropriate temperature before driving the OLED, the light output can be increased until the saturated power density is reached, without damaging the OLED. Here, the saturated power density refers to the maximum power density that longer increases, even when a greater power is applied to the OLED.

Additionally, generally, the more power applied to the OLED, the higher the temperature of the OLED increases. In contrast, if the OLED is cooled to an appropriate temperature before driving the OLED, the temperature of the OLED will not rise above protein denaturation temperature (for example, 40°C to 42°C or higher) or body temperature (for example, 36°C to 37.5°C) even when the OLED outputs light with the saturation power density.

Therefore, the control unit 40 controls such that the OLED is driven after the OLED is cooled to a cooling temperature whereby the OLED 10 can output light with a saturated power density without malfunction, more preferably, to a cooling temperature that ensures the temperature of the OLED does not rise to a higher temperature than protein denaturation temperature or body temperature even when the applied power is increased to output the light with the saturated power density.

In the OLED device according to one embodiment of the present disclosure, the preset cooling temperature is a temperature lower than room temperature by 5°C or more, preferably 5°C or below, 0°C to 5°C, or -15°C to 5°C, and more preferably, around 0°C or 0°C or below (-15°C to 0°C).

Additionally, the control unit 40 may control such that power is applied to the OLED within a preset maximum power range. Here, the maximum power is the power at which light with a saturated power density can be output from the OLED when applied to the OLED.

Furthermore, if the OLED is cooled to an appropriate temperature before driving the OLED, even when light with a saturated power density is output from the OLED, the temperature will not rise above the temperature at which protein denaturation can occur (for example, 40°C to 42°C) or above body temperature (for example, 36°C to 37.5°C). Therefore, the OLED device according to one embodiment of the present disclosure may preferably be used as a phototherapy device.

Meanwhile, due to internal or external factors, the temperature of the OLED may rise to a temperature where protein denaturation can occur. When the temperature measured by the temperature sensors 30, 31 reaches a preset maximum temperature (for example, the temperature at which protein denaturation can occur), the control unit 40 may control to lower the power applied to the OLED or to stop the driving of the OLED until the temperature decreases.

Additionally, when driving the OLED, if the temperature of the OLED does not exceed 20°C, and more preferably if the temperature of the OLED is 0°C to 5°C, the temperature of the skin located near the OLED can be cooled to 10°C to 15°C, making it effective in improving pruritus. Therefore, when improvement of pruritus is needed, the control unit may control the driving of the OLED such that the temperature measured by the temperature sensor 30, 31 reaches the preset temperature for improving pruritus (20°C or below, and more preferably 0°C to 5°C).

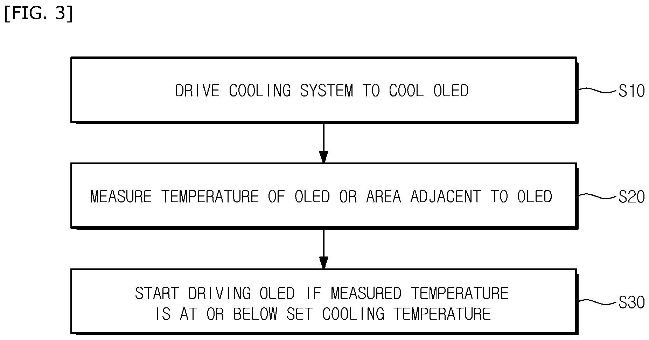

The driving method of the OLED device according to one embodiment of the present disclosure as aforementioned will be explained again with reference to FIG. 3 as follows.

When the driving of the OLED device according to one embodiment of the present disclosure starts, the control unit 40 drives the cooling system 20 to cool the OLED 10 (S10).

Through the cooling in step S10, the OLED 10 at room temperature (for example, 25°C) is cooled to a temperature lower than room temperature.

At this time, the temperature sensor 30 measures the temperature of the OLED or an area adjacent to the OLED (S20).

The temperature measured in step S20 is transferred to the control unit 40. The temperature sensor 30 may periodically measure the temperature of the OLED or the area adjacent to the OLED and send the measured temperature to the control unit.

The control unit 40 determines whether the measured temperature from the temperature sensor is below or equal to the preset cooling temperature, and if the measured temperature is below or equal to the preset cooling temperature, it drives the OLED (S30).

Here, the preset cooling temperature is the temperature that allows light with a saturated power density to be output without malfunction, when the OLED is driven after cooling the OLED to the cooling temperature. Furthermore, the cooling temperature is the temperature that ensures that the temperature measured by the temperature sensor when the OLED outputs light with a saturated power density is not heated to a temperature above the room temperature or protein denaturation temperature. The preset cooling temperature with these characteristics may be, for example, 5°C or below, and more preferably 0°C.

In addition, when the OLED device according to one embodiment of the present disclosure is a phototherapy device, especially when the OLED includes a light conversion film, it is preferable to ensure that light with sufficient power density for phototherapy is output.

Therefore, in driving the OLED, the control unit 40 may increase the applied power within the maximum power range that allows the OLED to output light with a saturated power density.

Meanwhile, when the OLED device according to one embodiment of the present disclosure is a phototherapy device, it is preferable that the temperature measured by the temperature sensor 30 does not rise above the preset maximum temperature, even when the control unit 40 applies the maximum power to the OLED. Here, the maximum temperature may be the temperature at which protein denaturation can occur (for example, 40°C to 42°C) or body temperature (for example, 36°C to 37.5°C).

Therefore, it is preferable to determine the cooling temperature in step S30 such that the temperature of the OLED does not rise above protein denaturation temperature or body temperature, even if the OLED reaches the saturation power density. Nonetheless, if the temperature measured by the temperature sensor reaches a preset maximum temperature, the control unit controls to lower the power supplied to the OLED or stop the driving of the OLED.

In addition, after driving the OLED to improve pruritus, the control unit controls so that the temperature measured by the temperature sensor maintains a preset temperature range for improving pruritus (20°C or below, and more preferably 0°C to 5°C), and if the temperature exceeds the preset temperature range, the control unit controls to reduce the power applied to the OLED or stop the driving of the OLED.

The OLED device 100 and driving method thereof according to one embodiment of the present disclosure described so far allow for the improvement of the electrical and optical characteristics of the OLED by driving the cooling system 20 before the control unit 40 drives the OLED 10 to cool the OLED to a preset cooling temperature or below, and then driving the OLED.

This OLED device according to one embodiment of the present disclosure may be a phototherapy device, and it is preferable that the phototherapy device is positioned close to the skin requiring treatment.

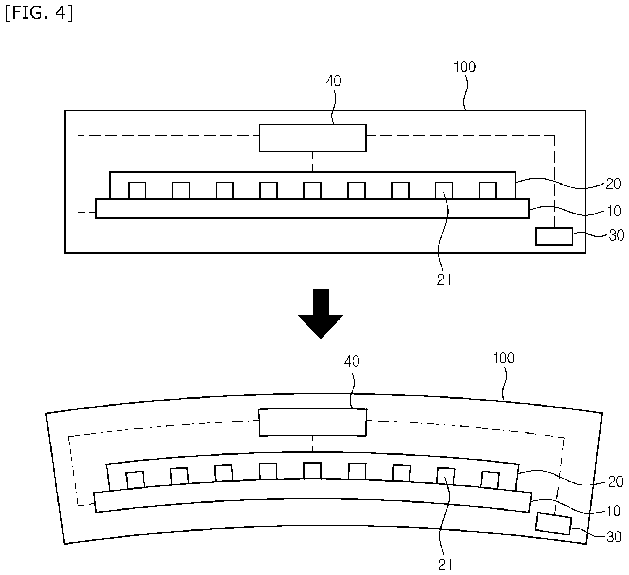

Accordingly, as shown in FIG. 4, it is preferable that the OLED device 100 according to one embodiment of the present disclosure is configured to have flexibility so that it can bend to conform to the curvature of the skin, such as the abdomen.

Specifically, the flexibility of the OLED device is achieved by using a flexible OLED as the OLED 10, and it is preferable that the cooling element 21, which lacks flexibility, is configured such that a plurality of small cooling elements are arranged at regular intervals on an upper part of the OLED 10, as shown in FIG. 4, so that the OLED device 100 as a whole maintains flexibility.

Meanwhile, when the phototherapy device is put in direct contact with the skin, heat generated by the light source may cause skin damage (protein denaturation). However, the OLED device according to one embodiment of the present disclosure is safe because the OLED is cooled to a low temperature before the OLED is driven, and even after the OLED is driven, the temperature of the OLED does not rise to the temperature (40°C) or above at which protein denaturation can occur.

Moreover, even when sufficient light for phototherapy is being output, the temperature of the OLED device 100 according to one embodiment of the present disclosure is maintained lower than the skin temperature, thereby improving pruritus of the skin that is directly contacted by the OLED device.

Furthermore, considering a further enhanced skin cooling function for the improvement of pruritus, it is preferable to control such that the temperature of the OLED does not exceed 20°C when the OLED is being driven, and if the temperature of the OLED is controlled to be 0°C to 5°C, the skin can be sufficiently locally cooled to 10°C to 15°C, which is more preferable.

In order to confirm the effects of the OLED device and driving method thereof according to one embodiment of the present disclosure, its stability as a phototherapy device, and the preferable cooling temperature of the OLED, the following tests were conducted.

### [Test 1]

As an OLED for Example 1, an OLED with the following characteristics was used:
- Phosphorescent OLED (Red OLED: ROLED)
- Glass/glass substrate
- A single-layer structure with one OLED unit element positioned between a substrate and a substrate.

The OLED of Example 1 was driven after being cooled to 0°C, and the temperature of the OLED and the light power density of the irradiance were measured while increasing the applied power.

As a Comparative Example 1, the same OLED as in Example 1 was used, but it was not cooled, but at room temperature (25°C), the temperature of the OLED and light power density of the irradiance were measured while increasing the applied power.

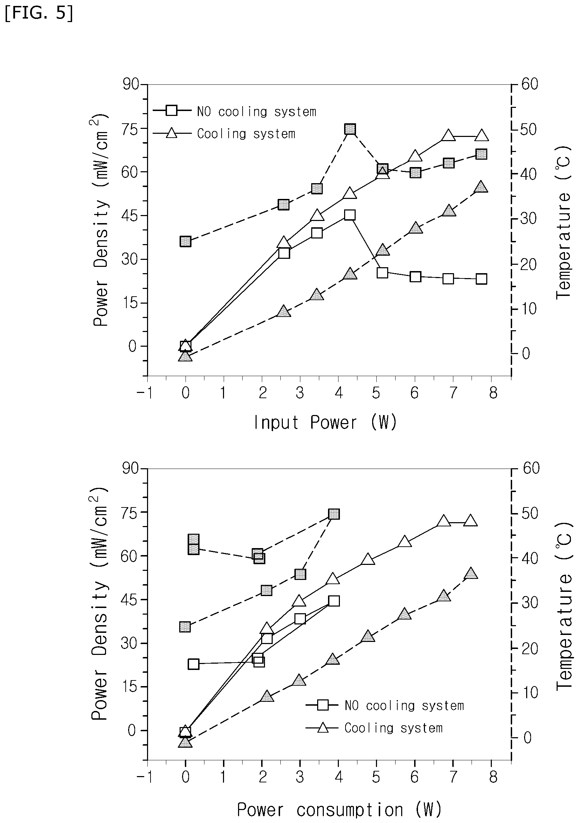

As a result, as shown in FIG. 5, the OLED of Example 1 showed a linear increase in the power density and temperature as the applied power was increased, showing a saturated power density of 72.2mW/cm², and up until reaching the saturated power density, the temperature of the OLED did not exceed 40°C, the safe temperature for skin treatment (protein denaturation temperature).

In contrast, in Comparative Example 1, after reaching a power density of 43mW/cm², which is lower than the saturated power density of 72.2mW/cm², the OLED was damaged, and thus, regardless of the applied amount of voltage and current, the output power, power density, and temperature all decreased.

Also, when the power density of 43 mW/cm², which is lower than the saturated power density of 72.2 mW/cm² in Example 1, was reached, the temperature already exceeded 40°C, which is the safe temperature for skin treatment.

Through this Test 1, it was confirmed that the OLED device according to one embodiment of the present disclosure not only has stability as a phototherapy device but also exhibits excellent electrical and optical characteristics.

Moreover, unlike Comparative Example 1, in Example 1, as the input power increased, the OLED's temperature and power density (irradiance) increased linearly, confirming that a user can easily adjust the treatment temperature and irradiance needed for the user by adjusting the input power.

### [Test 2]

As Comparative Example 2, the OLED of Example 1 was driven without cooling and at room temperature (25°C), simultaneously with the cooling system.

The light power density (irradiance) was measured while increasing the applied power, and the light power density was also measured as the power consumption increased.

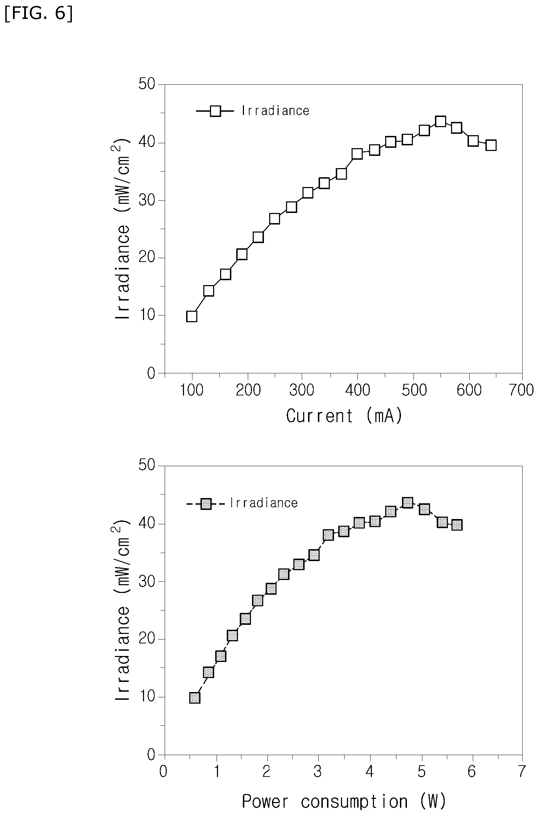

As a result, as shown in FIG. 6, it was confirmed that after reaching a power density of 45 mW/cm² at a power consumption of 4.75 W, a phenomenon of light quenching occurred, where both the irradiance and light power density decreased despite applying greater power.

Although this Comparative Example 2 showed a somewhat higher maximum power density compared to Comparative Example 1, which did not use a cooling system, it was confirmed that when compared to Example 1 which exhibited the saturated power density of 72.2 mW/cm², the electrical and optical characteristics of the OLED improved further when the OLED was cooled using the cooling system before being driven, even when the cooling system and OLED were driven together. This indicates that, although light quenching phenomenon typically occurs in light elements due to heat generation when excessive power is applied, cooling the OLED with the cooling system and then driving it allows for a continuous increase in light output as the applied power increases, due to coupling in the OLED emission layer.

### [Test 3]

The OLED of Example 1 was cooled to 0°C, 5°C, 10°C, 15°C, and 20°C, respectively, and then the temperature and light power density of the OLED were measured while increasing the applied power (i.e., the starting temperature for driving the OLED was set to 0°C, 5°C, 10°C, 15°C, and 20°C).

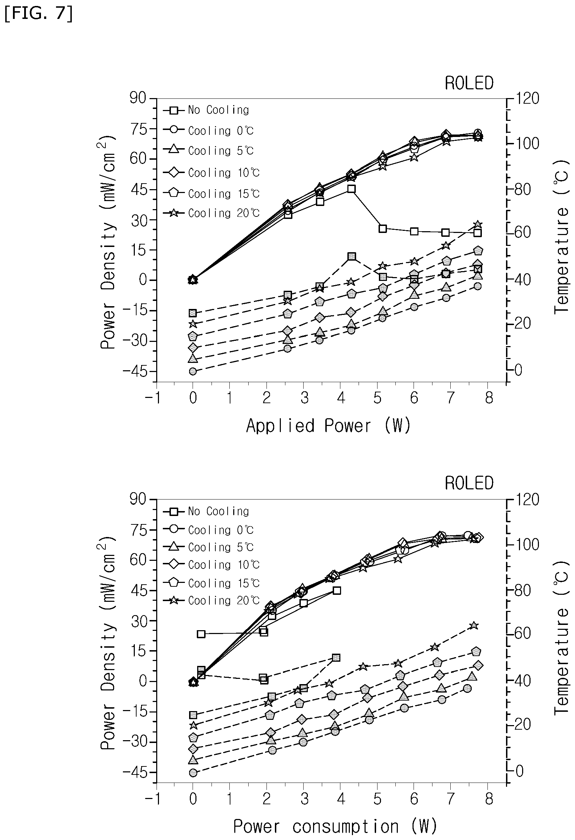

As a result, as shown in FIG. 7, it was confirmed that, in all cooling temperatures, the OLED's temperature and power density (irradiance) increased linearly with the increase in applied power. However, when the starting temperatures were set to 10°C, 15°C, or 20°C, the OLED's temperature exceeded 40°C when it reached the saturated power density.

Therefore, when the OLED device according to one embodiment of the present disclosure is a phototherapy device, and an OLED such as Example 1 is used, it is preferable that the cooling temperature is set to 5°C or lower.

Furthermore, after cooling the OLED of Example 1 to 0°C, 5°C, or 10°C, even when the OLED was driven not to exceed 20°C, light with a power density of approximately 40mW/cm² to 50mW/cm² was output. Thus, it can be confirmed that the OLED device according to one embodiment of the present disclosure can improve skin pruritus while simultaneously providing phototherapy.

Furthermore, when lowering the cooling temperature below 0°C (specifically -15°C to 0°C), it becomes possible to output light with sufficient power density even when the OLED is driven at a temperature of 0°C to 5°C, making it more desirable for improving pruritus.

### [Test 4]

As an OLED for Example 2, an OLED with the following characteristics was used:
- Phosphorescent OLED (Red OLED: ROLED)
- Metal/glass substrate (the substrate at the cooling system side is metal)
- A tandem structure with two OLED unit elements stacked between a substrate and a substrate

Using the OLED of Example 2, after the OLED was cooled to 0°C, 5°C, 10°C, 15°C, and 20°C, respectively, the OLED's temperature and light power density of irradiance were measured while increasing the applied power, in the same manner as in Test 3.

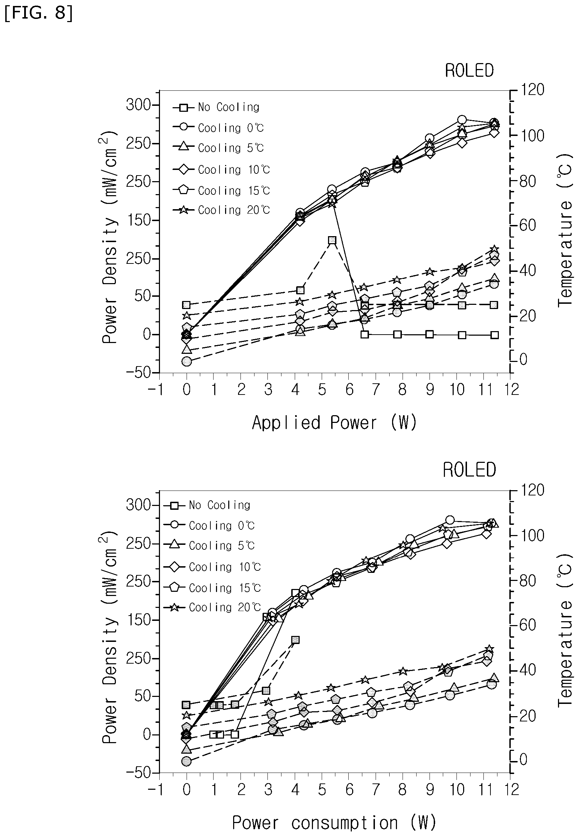

As a result, as shown in FIG. 8, in all cooling temperatures, the OLED's temperature and power density (irradiance) increased linearly with the increase of applied power, and a higher saturated power density (281mW/cm²) was measured compared to when the OLED of Example 1 was used.

This result is believed to be due to the use of a tandem structure OLED, which can output light with a higher power density, and also the use of a metal substrate, which allows a larger power to be applied without overheating the OLED.

Additionally, although the OLED of Example 2 outputs light with a higher saturated power density compared to the OLED of Example 1, the OLED temperature was maintained lower.

Therefore, when high irradiance (light power density) is required, it is preferable to use an OLED with a metal/glass substrate rather than a glass/glass substrate OLED.

Moreover, instead of a metal substrate, an OLED with a metal film layer capable of improving heat transfer, such as a plastic substrate, may also be used.

In this regard, users can adjust the electrical and optical characteristics of the OLED by choosing any one of the OLED encapsulation types, such as glass/glass, glass/metal, metal/plastic, and plastic/plastic etc.

Meanwhile, when the OLED of Example 2 was cooled to 0°C or 5°C, the OLED's temperature did not exceed body temperature when it reached the saturated power density.

Also, even when the OLED was driven not to exceed the temperature of 20°C, light with sufficient power density for phototherapy was output.

Therefore, when the OLED device according to one embodiment of the present disclosure is used as a phototherapy device and the OLED of Example 2 is used, it is preferable that the cooling temperature is 5°C or lower. In this case, even when the saturated power density is reached, not only is the temperature maintained lower than body temperature, but also light with sufficient power density for phototherapy is output even when driven not to exceed 20°C, making it effective for treating skin pruritus.

Furthermore, considering the skin that will be adjacent to the OLED 10, it is preferable for the preset temperature range for starting driving the OLED to be between -15°C and 5°C, and when the cooling temperature is between -15°C and 0°C, even when the OLED is driven at 0°C to 5°C not to exceed 5°C, light with sufficient power density can be output.

### [Test 5]

As an OLED for Example 3, an OLED with the following characteristics was used:
- Fluorescent blue OLED (BOLED)
- Metal/glass substrate
- A tandem structure with two OLED unit elements stacked between a substrate and a substrate

The OLED of Example 3 was cooled to 0°C, 5°C, 10°C, 15°C, and 20°C, respectively, and the OLED's temperature and light power density of irradiance were measured while increasing the applied power.

As Comparative Example 3, the same OLED as in Example 3 was used without cooling, at room temperature (25°C), and the OLED's temperature and light power density of irradiance were measured while increasing the applied power.

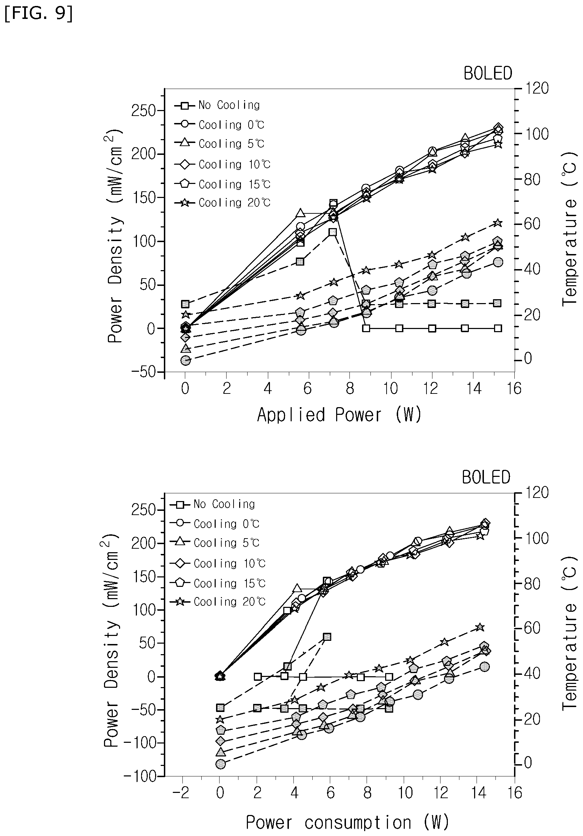

As a result, as shown in FIG. 9, in the case of Comparative Example 3, after reaching a power density of 142.96 mW/cm², the OLED was damaged, resulting in decreases in output power, power density, and temperature regardless of the amount of applied voltage and current. Furthermore, at the maximum power density, the temperature of the OLED significantly exceeded 40°C, reaching 56.8°C.

In contrast, in the OLED of Example 3, it was confirmed that as the applied power increased, the temperature and power density (irradiance) of the OLED increased linearly until reaching a saturated power density of 230 mW/cm² at all starting temperatures.

In particular, it was confirmed that the OLED of Example 3, despite being a fluorescent OLED, can enhance luminous efficiency through the method of having a cooling system and driving the OLED after cooling, as in the present disclosure.

In other words, compared to the phosphorescent OLED, which has an efficiency close to 100% in converting electrical energy into light, it can be seen that the present disclosure is highly effective in increasing the light conversion efficiency of a fluorescent OLED, which has an efficiency of around 25%.

### [Test 6]

The OLED of Example 1 was driven continuously for a total of 70 minutes, maintaining the applied power so that a saturated power density is output, and the changes in light irradiance and current were measured every 10 minutes.

Additionally, the OLED of Example 1 was operated continuously for 12 hours, maintaining the applied power so that the saturation power density is output, and the changes in irradiance and current were measured.

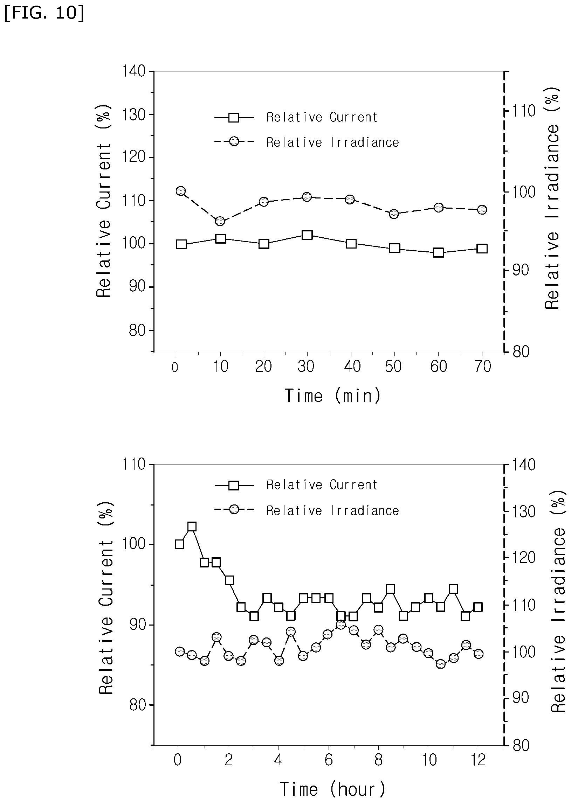

As a result, as shown in FIG. 10, during the continuous operation for 70 minutes, the relative current variation was between 97.8% to 100%, and the relative irradiance range was 96.2% to 100%, and during the 12-hour continuous operation, the relative irradiance was 97.3% to 105.8%, while the relative current stabilized in the range between 91% to 93.3%.

Through these tests, it was confirmed that the OLED device according to one embodiment of the present disclosure maintains stable electrical and optical characteristics even during long-duration continuous operation.

So far, the OLED device with cooling system and driving method thereof according to the embodiments of the present disclosure have been described in detail in a limited manner with reference to specific examples. However, the present disclosure is not limited to these specific embodiments, and it should be understood that various modifications and changes can be made without departing from the spirit and scope of the disclosure as defined in the claims.

## Claims

1. An OLED device, comprising:
an OLED (10);
a cooling system (20) comprising a cooling element (21) for cooling the OLED 10;
a temperature sensor (30, 31) for measuring a temperature of the OLED or of an area adjacent to the OLED; and
a control unit (40) for controlling driving of the OLED and of the cooling system,
**characterized in that** the control unit (40) drives the cooling system prior to driving the OLED, and controls such that the OLED starts to be driven when the temperature measured by the temperature sensor is at or below a preset cooling temperature.

2. The OLED device according to claim 1,
**characterized in that** the OLED (10) is a flexible OLED, and the cooling system (20) comprises a plurality of cooling elements arranged to be spaced apart from each other.

3. The OLED device according to claim 1 or claim 2,
**characterized in that** the cooling element (21) is a Peltier element, and the cooling system further comprises a heat sink and a fan for dissipating heat generated in the cooling element.

4. The OLED device according to any one of claims 1 to 3,
**characterized in that** the OLED (10) comprises one OLED unit element or a stack of a plurality of OLED unit elements between a substrate located at the cooling element's side and a substrate located at a light-emitting surface's side, and the upper substrate is a metal substrate or a substrate having a metal film layer.

5. The OLED device according to any one of claims 1 to 4,
**characterized in that** the cooling temperature is a temperature at which light with a saturated power density can be output without malfunction when the OLED 10 is driven after being cooled to the cooling temperature.

6. The OLED device according to any one of claims 1 to 5,
**characterized in that** the cooling temperature is a temperature that ensures the temperature measured by the temperature sensor when the OLED (10) outputs the light with the saturated power density is not heated to a temperature above body temperature or protein denaturation temperature.

7. The OLED device according to any one of claims 1 to 6,
**characterized in that** the cooling temperature is 5°C or below, or 0°C or below.

8. The OLED device according to any one of claims 1 to 7,
**characterized in that** the control unit (40) applies power to the OLED within a preset maximum power range, and the maximum power is a power at which the OLED outputs the light with the saturated power density when applied to the OLED.

9. The OLED device according to any one of claims 1 to 8,
**characterized in that** the control unit (40) controls to lower the power applied to the OLED or stop the driving of the OLED if the temperature measured by the temperature sensor is a higher temperature than body temperature or protein denaturation temperature.

10. The OLED device according to any one of claims 1 to 9,
**characterized in that** the cooling temperature is -15°C to 0°C, and the control unit (40) controls such that the temperature measured by the temperature sensor (30, 31) after the OLED (10) is driven maintains a range of 0°C to 5°C.

11. A driving method of an OLED device, **characterized by** comprising:
driving a cooling system (20) to cool an OLED;
measuring a temperature of the OLED or of an area adjacent to the OLED with a temperature sensor (30, 31); and
starting to drive the OLED if the measured temperature is at or below a preset cooling temperature.

12. The driving method of the OLED device according to claim 11,
**characterized in that** the cooling temperature is a temperature at which light with a saturated power density can be output without malfunction when the OLED is driven after being cooled to the cooling temperature, or a temperature that ensures the temperature measured by the temperature sensor (30, 31) when the OLED outputs the light with the saturated power density is not heated to a temperature above body temperature or protein denaturation temperature.

13. The driving method of the OLED device according to claim 11 or claim 12,
**characterized in that** the cooling temperature is 5°C or below, or 0°C or below.

14. The driving method of the OLED device according to any one of claims 11 to 13,
**characterized by** further comprising lowering the power applied to the OLED or stopping the driving of the OLED if the temperature measured by the temperature sensor (30, 31) after the OLED (10) is driven is higher than body temperature or protein denaturation temperature.

15. The driving method of the OLED device according to any one of claims 11 to 14,
**characterized in that** the cooling temperature is -15°C to 0°C, and further comprises controlling such that the temperature measured by the temperature sensor (30, 31) after the OLED (10) is driven maintains a range of 0°C to 5°C.
